# EUROPEAN PATENT SPECIFICATION

(11) **EP 4 262 941 B1**
(45) Date of publication and mention of the grant of the patent: **27.11.2024**
(21) Application number: 21819235.9
(22) Date of filing: 01.12.2021
(51) Int. Cl.: A61M 15/00, A24F 40/20, A24F 40/05, A24F 40/42, A24F 42/20, A24F 42/60, A61M 15/06

(54) **INHALER ARTICLE AND CAPSULE FOR USE WITH THE SAME**
INHALATIONSARTIKEL UND KAPSEL ZUR VERWENDUNG DAMIT
ARTICLE D'INHALATEUR ET CAPSULE À UTILISER AVEC CELUI-CI

(30) Priority: 18.12.2020 EP 20215318
(43) Date of publication of application: 25.10.2023
(73) Proprietor: Philip Morris Products S.A., 2000 Neuchâtel (CH)
(72) Inventor: CAMPITELLI, Gennaro, 2000 Neuchâtel (CH); CONSOLANTE, Antonio, 40069 Bologna (IT)
(74) Representative: Abitz & Partner
(86) International application number: PCT/IB2021/061197
(87) International publication number: WO 2022/130091

(56) References cited:
- US-A1- 2010 300 439
- US-A1- 2015 136 131

## Description

The present disclosure relates to a dry powder capsule with side piercing for use in an inhaler article, and to inhaler articles comprising the dry powder capsule.

Dry powder inhalers used to dispense powdered medicaments often strive to provide an entire dry powder dose in a single breath. Such dry powder inhalers are often complex in their design and may involve moving parts. In addition, these complex dry powder inhalers are difficult to produce and assemble at high speeds.

Dry powder inhalers that are constructed to provide dry powder particles to the lungs at inhalation (air flow) rates that are within conventional smoking regime inhalation (air flow) rates may be designed with a linear airflow path between the powder receptacle and the outlet. Dry powder inhalers may contain inhalable powder in a capsule that may be pierced to access the powder. However, loose powder may sometimes unintentionally fall out of such inhalers if the inhaler is inverted or otherwise manipulated in a non-upright position.

US20150136131A1 relates to a system composed of an inhaler and a capsule. US20100300439A1 relates to a nasal delivery device for and method of delivering particulate substance to the nasal airway of a subject.

It is desirable to provide a dry powder inhaler that mitigates unintentional powder leakage from the inhaler. It is desirable to provide a dry powder inhaler that mitigates unintentional powder leakage from a dry powder capsule disposed in the inhaler. It is desirable to provide a capsule for an inhaler that is pierced such that dry powder may be inhaled when desired but that reduces the amount of loose powder within the inhaler (outside of the capsule) when the inhaler is not actively being used. It is desirable to provide an inhaler with a simple design that is easy to manufacture and assemble and that provides the benefit of reduced powder leakage.

According to an embodiment of the present disclosure, an inhaler article comprises a body with an upstream end and a downstream end, a capsule receptacle, and an airflow path extending from the capsule receptacle to an opening at the downstream end. The downstream end is the mouthpiece end of the inhaler article. A capsule is disposed inside the capsule receptacle. The capsule comprises a first end and a second end, and a tubular side wall extending between the first and second ends. The capsule comprises only a single aperture. The single aperture extends through the tubular side wall of the capsule. The capsule further comprises an inhalable powder disposed inside the capsule.

Advantageously, providing only a single aperture in the capsule positioned at the side of the capsule (as opposed to one of the ends) reduces unintentional loss of dry powder from the capsule. It has been found that a single aperture at the side of the capsule allows inhalation of the dry powder from inside the capsule at air flow rates that are within conventional smoking regime inhalation (air flow) rates. The capsule allows use of a simple inhaler design while mitigating powder loss from the inhaler. By using this technique to mitigate powder leakage, the powder remains inside the capsule until it is used or inhaled. Mitigating powder leakage by placing the single aperture on the side of the capsule does not require any changes to the inhaler article itself. Placing the single aperture on the side of the capsule does not require any changes to the capsule as it is manufactured. Forming the single aperture on the side of the capsule may be performed by user as the capsule and inhaler is prepared for use.

The capsule may comprise a first end cap at the first end and a second end cap at the second end. Each of the first and second end caps of the capsule may comprise a hemisphere. The first end of the capsule may be oriented toward the upstream end of the body. The second end may be oriented toward the downstream end of the body. In some embodiments, the single aperture extends through the tubular side wall and is disposed closer to the first end than the second end. In some embodiments, the single aperture extends through the tubular side wall and is disposed closer to the upstream end than the downstream end. According to an embodiment, the first and second end caps do not include any apertures.

Advantageously, positioning the aperture closer to the upstream end provides a greater delivery of powder than positioning the aperture closer to the downstream end of the capsule. Surprisingly, it has been found that positioning the aperture on the side, closer to the upstream end provides the same or nearly the same amount of powder delivery per inhalation (puff) as positioning the aperture at the upstream end of the capsule. Advantageously, positioning the single aperture on the side wall mitigates unintentional loss of dry powder from the capsule.

The position of the aperture may be determined from a midpoint of the capsule measured along a longitudinal axis of the capsule. The midpoint may be determined by dividing the capsule side wall length in half. The distance of the aperture from the midpoint may be determined as the actual measured distance, or as a percentage of the length toward one end or the other (for example, "% toward first end") calculated by dividing the distance of the aperture from the midpoint by the half length of the capsule side wall. The single aperture may be positioned 0 % to 100 % toward the first end. The single aperture may be positioned 10 % or more, 25 % or more, 50 % or more, or 75 % or more toward the first end. The actual distance of the aperture from the midpoint may be selected based on the size of the capsule. In some cases, the capsule is a size 1, size 2, size 3, size 4, or size 5 capsule, preferably a size 3. The capsule may have a length of about 11 mm, about 14 mm, about 16 mm, about 18 mm, or about 19 mm. The capsule side wall may have a length of about 8 mm, about 10 mm, about 11 mm, about 12 mm, or about 14 mm. The single aperture may be positioned 1 mm or more, 3 mm or more, or 5 mm or more toward the first end of the capsule, measured from the midpoint. The single aperture may be positioned 7 m or less, 6 mm or less, or 5 mm or less toward the first end of the capsule, measured from the midpoint. The single aperture may be within 2 mm, within 4 mm, within 6 mm, or within 10 mm of the first end as measured from the first end along a longitudinal axis of the capsule. The single aperture may be within 1 mm, within 2 mm, within 4 mm, within 6 mm, or within 10 mm of the first end cap as measured from the upstream end of the sidewall along a longitudinal axis of the capsule.

Preferably, the capsule receptacle has dimensions that cause the capsule to maintain its orientation within the capsule receptacle. For example, the capsule receptacle may have a width that causes the capsule to remain oriented such that the first end of the capsule points toward the upstream end of the body. A suitable width may be selected based on the width and length of the capsule so that the capsule has some room to move (e.g., vibrate or rotate) inside the capsule receptacle but does not have enough room to turn end-over-end. For example, the capsule may have a length and the capsule receptacle may have a width that is less than the length of the capsule. The capsule receptacle width may also be determined based on the width or diameter of the capsule. For example, the capsule receptacle may have a width (diameter) that is 5 % to 25 % greater than the width (diameter) of the capsule. The diameter of the capsule receptacle may be 8 % to 22 % greater or from 10 % to 20 % greater than the diameter of the capsule. The capsule may be a two-part capsule, where one part has a diameter slightly larger than the other so that the two parts can telescopically fit together. The single aperture may be disposed along the side wall of the capsule in an area where the diameter of the capsule receptacle is only up to 20 % greater, or only up to 15 % greater, than the diameter of the capsule. If the capsule is positioned in the center of the capsule receptacle, there may be less than 2 mm, less than 1.5 mm, less than 1.2 mm, less than 1 mm, less than 0.8 mm, or less than 0.5 mm space between the capsule side wall and the wall of the capsule receptacle. In some cases, the capsule receptacle is sized for a size 3 capsule and may have an inner diameter in a range from about 6 mm to about 7 mm, or about 6.5 mm to about 6.7 mm. The capsule receptacle may have a length in a range from about 15 mm to about 30 mm, or from about 18 mm to about 25 mm. The capsule receptacle may define a cylindrical or substantially cylindrical space constructed to contain the capsule. The capsule receptacle may have a substantially uniform or uniform diameter along the length of the capsule receptacle.

The single aperture may have a suitable size that allows a desired amount of powder to exit the capsule upon inhalation. For example, the single aperture may have a diameter of 0.5 mm or greater, 0.6 m or greater, or 0.7 mm or greater. The single aperture may have a diameter of 1.5 mm or less, 1.2 mm or less, or 1.0 mm or less. The single aperture may have a diameter of 0.5 mm to 1.2 mm, 0.7 mm to 1.0 mm, or about 0.8 mm. The single aperture may have an area of 0.2 mm² or greater, 0.3 mm² or greater, or 0.4 mm² or greater. The single aperture may have an area of 1.8 mm² or less, 1.5 mm² or less, 1.2 mm² or less, 1.0 mm² or less, or 0.8 mm² or less. The single aperture may have an area of 0.2 mm² to 1.5 mm² or 0.4 mm² to 0.8 mm².

The inhaler article may be used for the inhalation of any desired dry powder. According to an embodiment, the capsule contains dry powder comprising particles containing one or more pharmaceutically active agents. Examples of pharmaceutically active agents include nicotine, anatabine, antiviral compounds such as acyclovir; anti-inflammatory compounds such as salicylic acid, aceclofenac, or ketoprofen; antidiabetic compounds such as metformin or glipizide; antihypertensive compounds such as oxprenolol; antiemetic compounds such as promethazine; antidepressant compounds such as seproxetine; anticoagulant compounds such as picotamide; bronchodilators such as clenbuterol; or anticancer compounds such as beta-lapachone. The pharmaceutically active agent may include a pharmaceutically acceptable salt of the agent. Suitable salts include, for example, a salt of lactic acid ("lactate"), tartaric acid ("tartrate" or "bitartrate"), aspartic acid ("aspartate"), pyruvic acid ("pyruvate"), citric acid ("citrate"), salicylic acid ("salicylate"), glutamic acid ("glutamate"), gentisic acid ("gentisate"), benzoic acid ("benzoate"), fumaric acid ("fumarate"), hydrochloric acid ("hydrochlorate"), alfa-resorcylic acid ("alfa-resorcylate"), beta-resorcylic acid ("beta-resorcylate"), oxalic acid ("oxalate"), p-anisic acid ("anisate"), glutaric acid ("glutarate"), and the like. In some cases, the capsule contains nicotine powder. For example, the capsule may contain a dry powder comprising nicotine salt. The dry powder may further contain other components, such as sugar or sugar alcohol, amino acid, flavorant, cough suppressant, or other pharmaceutically acceptable ingredients that are suitable for use in inhalable powders. In one embodiment, the capsule contains nicotine powder comprising nicotine particles, where the nicotine particles comprise nicotine salt, sugar or sugar alcohol, and amino acid. The capsule may further comprise flavor particles, cough suppressant particles, or both flavor and cough suppressant particles. Flavor and cough suppressant particles are collectively referred to here as flavor particles. The capsule may contain an inhalable powder comprising particles with MMAD particle size in the range of 0.5 µm to 10 µm, or 0.5 µm to 5 µm. In one embodiment, the capsule contains nicotine powder comprising nicotine particles, where the nicotine particles have MMAD particle size in the range of 0.5 µm to 10 µm, or 0.5 µm to 5 µm. The capsule may further contain flavor particles having MMAD particle size of 10 µm or greater, 20 µm or greater, or 40 µm or greater. The flavor particles may have MMAD particle size of 200 µm or less, 150 µm or less, or 120 µm or less. The flavor particles may have MMAD particle size of 20 µm to 200 µm or from 40 µm to 120 µm. In some embodiments, the capsule contains nicotine particles having MMAD particle size in the range of 0.5 µm to 10 µm, or 0.5 µm to 5 µm and flavor particles having MMAD particle size of 20 µm to 200 µm or from 50 µm to 150 µm.

The capsule may contain a predetermined amount of nicotine particles and optional flavor particles. The capsule may contain enough nicotine particles to provide at least 2 inhalations or "puffs," or at least 5 inhalations or "puffs," or at least 10 inhalations or "puffs." The capsule may contain enough nicotine particles to provide from 5 to 50 inhalations or "puffs," or from 10 to 30 inhalations or "puffs." Each inhalation or "puff" may deliver from 0.1 mg to 3 mg of nicotine particles, from 0.2 mg to 2 mg of nicotine particles, or about 1 mg of nicotine particles, to the lungs of the user.

The nicotine particles may have any useful concentration of nicotine based on the particular formulation employed. The nicotine particles may have at least about 1 wt-% nicotine up to about 30 wt-% nicotine, or from about 2 wt-% to about 25 wt-% nicotine, or from about 3 wt-% to about 20 wt-% nicotine, or from about 4 wt-% to about 15 wt-% nicotine, or from about 5 wt-% to about 13 wt-% nicotine.

The capsule may hold or contain 5 mg or more, or 10 mg or more of nicotine particles. The capsule may hold or contain 900 mg or less, 600 mg or less, 300 mg or less, or 150 mg or less of nicotine particles. The capsule may hold or contain from 5 mg to 300 mg or from 10 mg to 200 mg of nicotine particles. When flavor particles are blended or combined with the nicotine particles in the capsule, the flavor particles may be present in an amount that provides the desired flavor to each inhalation or "puff" delivered to the user. The capsule may hold or contain 5 mg or more, or 10 mg or more of dry powder (also referred to as a powder system). The capsule may hold or contain 900 mg or less, 600 mg or less, 300 mg or less, or 150 mg or less of dry powder. The capsule may hold or contain from 5 mg to 300 mg, from 10 mg to 200 mg, or from 25 mg to 100 mg of a dry powder. The nicotine particles may make up 40 wt-% or more, 60 wt-% or more, or 80 wt-% or more of the dry powder.

The dry powder (powder system) may have a mean diameter of 60 µm or less, or in a range from 1 µm to 40 µm, or from 1.5 µm to 25 µm. The mean diameter refers to the mean diameter per mass as measured by laser diffraction, laser diffusion, or an electronic microscope, preferably by laser diffraction.

The nicotine in the powder system or nicotine particles may be a pharmaceutically acceptable free-base nicotine, or nicotine salt or nicotine salt hydrate. Useful nicotine salts or nicotine salt hydrates include nicotine pyruvate, nicotine citrate, nicotine aspartate, nicotine lactate, nicotine bitartrate, nicotine salicylate, nicotine fumarate, nicotine mono-pyruvate, nicotine glutamate or nicotine hydrochloride, for example. The nicotine particles preferably include an amino acid. Preferably the amino acid may be leucine such as L-leucine. Providing an amino acid, such as L-leucine, with the particles comprising nicotine may reduce adhesion forces of the particles and may reduce attraction between nicotine particles, thus reducing agglomeration of nicotine particles and adherence of the nicotine particles to surfaces. Similarly, adhesion forces to particles comprising flavor may also be reduced. The powder system may be a free-flowing material and possess a stable relative particle size of each powder component even when the nicotine particles and the flavor particles are combined.

The particles comprising flavor may include a compound to reduce adhesion forces or surface energy and resulting agglomeration. The flavor particle may be surface modified with an adhesion reducing compound to form a coated flavor particle. One preferred adhesion reducing compound is magnesium stearate. Providing an adhesion reducing compound such as magnesium stearate with the flavor particle, especially coating the flavor particle, may reduce adhesion forces of the particles comprising flavor and may reduce attraction between flavor particles and thus reduce agglomeration of flavor particles. Agglomeration of flavor particles with nicotine particles may also be reduced. The powder system described herein thus may possess a stable relative particle size of the particles comprising nicotine and the particles comprising flavor even when the nicotine particles and the flavor particles are combined. The powder system preferably may be free flowing.

Conventional formulations for dry powder inhalation contain carrier particles that serve to increase the fluidization of the active particles since the active particles may be too small to be influenced by simple airflow though the inhaler. The powder system may comprise carrier particles. These carrier particles may be a saccharide such as lactose or mannitol and may have a particle size greater than 50 µm. The carrier particles may be utilized to improve dose uniformity by acting as a diluent or bulking agent in a formulation. In some embodiments, the capsule contains a powder system that is carrier-free or substantially free of a saccharide particle such as lactose or mannitol. By carrier-free, it is meant that the powder system does not contain separate carrier particles (e.g., saccharide particles) in addition to the nicotine particles (which may contain sugar or sugar alcohol) and the optional flavor particles. Being carrier-free or substantially free of a saccharide such as lactose or mannitol may allow the nicotine and to be inhaled and delivered to the user's lungs at inhalation or airflow rates that are similar to typical smoking regime inhalation or airflow rates.

The capsule may be made of any suitable material. For example, the capsule may be made of a polymeric material, gelatin, or any other suitable material for making fillable hard capsules. In one embodiment, the capsule is made from polymeric material, preferably hydroxypropyl-methylcellulose ("HPMC"). For example, the capsule may be a size 1 HPMC capsule. The capsule may be a size 2 HPMC capsule. The capsule may be a size 3 HPMC capsule. The capsule may be a size 4 HPMC capsule. The capsule may be a size 1 gelatin capsule. The capsule may be a size 2 gelatin capsule. The capsule may be a size 3 gelatin capsule. The capsule may be a size 4 gelatin capsule.

The inhaler article of the present disclosure allows powder to be inhaled at inhalation (air flow) rates that are within conventional smoking regime inhalation (air flow) rates. According to an aspect of the present disclosure, the inhaler article comprises an inhaler body with a tubular side wall defining an interior and a longitudinal center axis. The interior forms a receptacle for housing the capsule containing inhalable powder. The inhaler article further comprises a mouthpiece element. The inhaler body and the mouthpiece have a simple design that is easy to manufacture and easy to assemble into an inhaler article. According to an embodiment, the mouthpiece may be simply inserted into a tubular inhaler body. The mouthpiece element extends along the longitudinal center axis from an upstream end to a downstream end. The upstream end of the mouthpiece element is received in the interior of the inhaler body. The mouthpiece element comprises an airflow channel extending through the mouthpiece element. The downstream end of the mouthpiece element may form or be disposed at the mouth end of the inhaler article. The upstream end of the mouthpiece element is inserted into or disposed inside the inhaler body. The upstream end of the mouthpiece element may form a downstream end of the capsule receptacle. The receptacle may extend from the upstream end of the inhaler body to the upstream end of the mouthpiece element. The inhaler body may have a closed upstream end. The upstream end of the inhaler body may be folded closed. For example, the upstream end of the inhaler body may be folded closed by a fan fold. The upstream end of the inhaler body may form the upstream end of the receptacle. The upstream end of the inhaler body may be opened prior to use, for example by an inhaler holder. The inhaler article may be designed to exhibit a desired resistance to draw ("RTD"). The inhaler article may have an RTD ranging from 30 to 200 mmWG.

The inhaler article comprises a body that defines the capsule receptacle and an airflow path extending from the capsule receptacle to an opening or outlet. The inhaler article body may be constructed of any suitable material. For example, inhaler article body may be constructed of cellulosic material, polymeric material, metal, or a combination thereof. According to an embodiment, the body is constructed of cellulosic material, preferably paper, paper board, or cardboard. In a preferred embodiment, the inhaler article comprises a tubular body made from paper, paper board, or cardboard. The upstream end of the tubular body made of paper, paper board, or cardboard may be folded closed, preferably by a fan fold. The closed end may be opened prior to use of the inhaler.

The inhaler article may further comprise a wrapper wrapped about at least a portion of the inhaler body. The wrapper may also be wrapped about at least a portion of the mouthpiece element. In some embodiments, the wrapper does not cover the entire length of the mouthpiece element such that a portion of the length of the mouthpiece element is left uncovered. The wrapper may be a paper wrapper, such as a cigarette wrapper or tipping paper.

Advantageously, an inhaler article made of cellulosic material is easy and inexpensive to manufacture, and is environmentally friendly and biodegradable.

The capsule may be sealed within the inhaler article prior to consumption. The capsule may be pre-loaded into the inhaler. The inhaler article, with the capsule contained inside the capsule receptacle, may be contained within a sealed or airtight container or bag. The inhaler article may include one or more peelable or removable seal layers to cover the one or more air inlet channels or the air outlet or mouthpiece of the inhaler article.

The inhaler article may be used with a holder. For example, the inhaler article may be configured for use with a holder that is capable of opening the closed upstream end of the inhaler article. The inhaler article may be configured for use with a holder that is capable of piercing a dry powder capsule housed in the receptacle. The inhaler article may be configured for use with a holder that provides an air inlet for the inhaler article. The holder may be constructed to activate the inhaler article by opening the closed end of the inhaler article. The holder may be constructed to activate the inhaler article by piercing the capsule with a piercing element. The holder may be constructed to release the particles contained inside the capsule and to enable the article to deliver the particles to a consumer. A plurality of inhaler articles may be combined with a holder to form a system or kit. A user may active and use one inhaler article and capsule at a time. A single holder may be utilized on 10 or more, or 25 or more, or 50 or more, or 100 or more inhaler articles to activate (puncture or pierce) a capsule contained within each inhaler article and to inhale the powder contained inside the capsule.

According to an embodiment, a method of preparing an inhaler article for use comprises using a piercing element to pierce a single aperture into the tubular side wall of the capsule. The capsule comprises a first end and a second end, and a tubular side wall extending between the first and second ends. The capsule may be disposed inside the capsule receptacle of the inhaler article such that the first end points toward the upstream end of the inhaler article. The capsule comprises an inhalable powder disposed inside the capsule. The method comprises forming a single aperture through the side wall of the capsule. The method may comprise forming the single aperture closer to the first end than the second end. The single aperture may be formed 10 % or more, 25 % or more, 50 % or more, or 75 % or more toward the first end, measured from a midpoint of the capsule. The actual distance of the aperture from the midpoint may be selected based on the size of the capsule. In some cases, the capsule is a size 1, size 2, size 3, size 4, or size 5 capsule, preferably a size 3. The capsule may have a length of about 11 mm, about 14 mm, about 16 mm, about 18 mm, or about 19 mm. The capsule side wall may have a length of about 8 mm, about 10 mm, about 11 mm, about 12 mm, or about 14 mm. The single aperture may be positioned 1 mm or more, 3 mm or more, or 5 mm or more toward the first end of the capsule, measured from the midpoint. The single aperture may be positioned 7 m or less, 6 mm or less, or 5 mm or less toward the first end of the capsule, measured from the midpoint. The single aperture may be within 2 mm, within 4 mm, within 6 mm, or within 10 mm of the first end as measured along a longitudinal axis of the capsule.

The method may comprise forming a single aperture in the capsule side wall, the single aperture having a diameter of 0.5 mm or greater, 0.6 m or greater, or 0.7 mm or greater. The single aperture may have a diameter of 1.5 mm or less, 1.2 mm or less, or 1.0 mm or less. The single aperture may have a diameter of 0.5 mm to 1.2 mm, 0.7 mm to 1.0 mm, or about 0.8 mm. The single aperture may have an area of 0.2 mm² or greater, 0.3 mm² or greater, or 0.4 mm² or greater. The single aperture may have an area of 1.8 mm² or less, 1.5 mm² or less, 1.2 mm² or less, 1.0 mm² or less, or 0.8 mm² or less. The single aperture may have an area of 0.2 mm² to 1.5 mm² or 0.4 mm² to 0.8 mm².

The inhaler may be less complex and have a simplified airflow path as compared to conventional dry powder inhalers. The inhaler may be constructed to provide a swirling air flow that causes the capsule to rotate about its longitudinal axis upon inhalation. Advantageously, rotation of the capsule within the inhaler more effectively aerosolizes the dry powder and may assist in maintaining a free-flowing powder. Thus, the inhaler article may not require the elevated inhalation rates typically utilized by conventional inhalers to deliver the nicotine particles described above deep into the lungs.

The inhaler article and the capsule may use a flow rate of 5 L/min or less, 3 L/min or less, 2 L/min or less, or 1.6 L/min or less. Preferably, the flow rate may be in a range from 1 L/min to 3 L/min or from 1.5 L/min to 2.5 L/min. Preferably, the inhalation rate or flow rate may be similar to that of Health Canada smoking regime, that is, 1.6 L/min.

The inhaler and capsule may be used by a consumer similar to smoking a conventional cigarette or vaping an electronic cigarette. Such smoking or vaping may be characterized by two steps: a first step during which a small volume containing the full amount of nicotine desired by the consumer is drawn into the mouth cavity, followed by a second step during which this small volume comprising the aerosol comprising the desired amount of nicotine is further diluted by fresh air and drawn deeper into the lungs. Both steps are controlled by the consumer. During the first inhalation step the consumer may determine the amount of nicotine to be inhaled. During the second step, the consumer may determine the volume for diluting the first volume to be drawn deeper into the lungs, maximizing the concentration of active agent delivered to the airway epithelial surface. This smoking mechanism is sometimes called "puff-inhale-exhale."

All scientific and technical terms used herein have meanings commonly used in the art unless otherwise specified. The definitions provided herein are to facilitate understanding of certain terms used frequently herein and are not meant to limit the scope of the present disclosure.

The term "nicotine" refers to nicotine and nicotine derivatives such as free-base nicotine, nicotine salts, and the like.

The term "flavorant" or "flavor" refers to organoleptic compounds, compositions, or materials that alter and are intended to alter the taste or aroma characteristics of nicotine during consumption or inhalation thereof.

The terms "upstream" and "downstream" refer to relative positions of elements of the holder, inhaler article and inhaler systems described in relation to the direction of inhalation air flow as it is drawn through the body of the holder, inhaler article and inhaler systems.

Unless otherwise specified, the term "particle size" as used here refers to mass median aerodynamic diameter (MMAD). MMAD is preferably measured with a cascade impactor.

The term "substantially" as used here has the same meaning as "significantly," and can be understood to modify the term that follows by at least about 90 %, at least about 95 %, or at least about 98 %.

The term "not substantially" as used here has the same meaning as "not significantly," and can be understood to have the inverse meaning of "substantially," i.e., modifying the term that follows by not more than 25 %, not more than 10 %, not more than 5 %, or not more than 2 %.

For the purpose of the present description and of the appended claims, except where otherwise indicated, all numbers expressing amounts, quantities, percentages, and so forth, are to be understood as being modified in all instances by the term "about." Also, all ranges include the maximum and minimum points disclosed and include any intermediate ranges therein, which may or may not be specifically enumerated herein. In this context, therefore, a number A is understood as A ± 5 % of A. Within this context, a number A may be considered to include numerical values that are within general standard error for the measurement of the property that the number A modifies. The number A, in some instances as used in the appended claims, may deviate by the percentages enumerated above provided that the amount by which A deviates does not materially affect the basic and novel characteristic(s) of the claimed invention. Also, all ranges include the maximum and minimum points disclosed and include any intermediate ranges therein, which may or may not be specifically enumerated herein.

Terms such as "a," "an," and "the" are not intended to refer to only a singular entity, but include the general class of which a specific example may be used for illustration.

The terms "a," "an," and "the" are used interchangeably with the term "at least one." The phrases "at least one of" and "comprises at least one of" followed by a list refers to any one of the items in the list and any combination of two or more items in the list.

As used here, the term "or" is generally employed in its usual sense including "and/or" unless the content clearly dictates otherwise. The term "and/or" means one or all of the listed elements or a combination of any two or more of the listed elements.

As used here, "have", "having", "include", "including", "comprise", "comprising" or the like are used in their open-ended sense, and generally mean "including, but not limited to." It will be understood that "consisting essentially of," "consisting of," and the like are subsumed in "comprising" and the like. As used herein, "consisting essentially of," as it relates to a composition, product, method or the like, means that the components of the composition, product, method or the like are limited to the enumerated components and any other components that do not materially affect the basic and novel characteristic(s) of the composition, product, method or the like.

The words "preferred" and "preferably" refer to embodiments that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the disclosure, including the claims.

The invention is defined in the claims.

Examples will now be further described with reference to the figures in which:
FIGURE 1A is a side view of a capsule according to an embodiment.
FIGURE 1B is a schematic side view of a capsule with a single aperture according to an embodiment.
FIGURE 2A a perspective view of an inhaler article according to an embodiment.
FIGURE 2B is a cross sectional side view of a capsule disposed in the inhaler article of FIGURE 2A according to an embodiment.
FIGURE 3 is a cross sectional side view of the inhaler article and capsule of FIGURE 2B in use with a holder.
FIGURE 4 is a partial cross sectional side view of the inhaler article and capsule of FIGURE 2B.

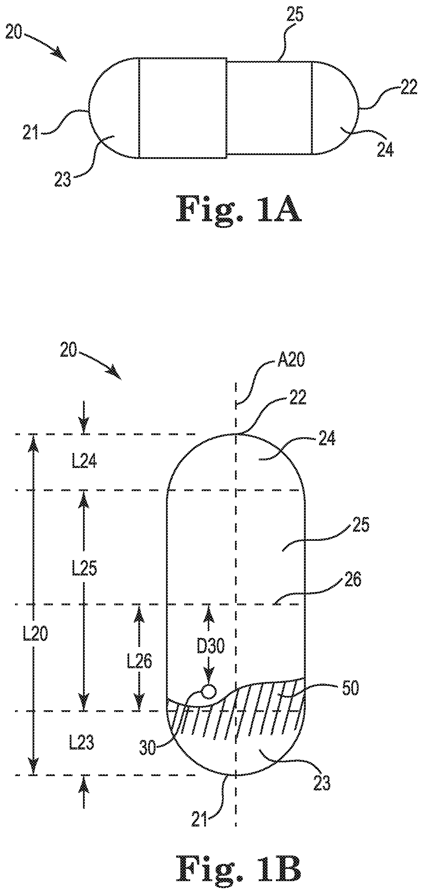

A capsule 20 containing inhalable powder 50 is shown in FIGURES 1A and 1B. The capsule 20 comprises a first end 21 and a second end 22. The capsule 20 may comprise a first end cap 23 at the first end 21 and a second end cap 24 at the second end 22. The capsule 20 comprises a tubular side wall 25 extending between the first and second ends 21, 22, or between the first and second end caps 23, 24. Each of the first and second end caps 23, 24 of the capsule may be shaped as a hemisphere, as shown. The capsule 20 comprises only a single aperture 30. The single aperture 30 extends through the tubular side wall 25 of the capsule 20.

In some embodiments, the single aperture 30 is disposed closer to the first end 21 than the second end 22. The distance of the aperture 30 may be determined from a midpoint 26 of the capsule, measured along a longitudinal axis A20 of the capsule 20. The capsule side wall 25 has a length 25, and the midpoint 26 defines a half length L26. The first and second end caps 23, 24 each have a length L23, L24, respectively. The total length L20 of the capsule 20 is the side wall length L25 plus the length L23 of the first end cap 23 and the length L24 of the second end cap.

The distance D30 of the aperture 30 from the midpoint 26 may be determined as the actual measured distance, or as a percentage of the length toward one end of the other (for example, "% toward first end") calculated as D30/L26. The single aperture 30 may be positioned 0 % to 100 % toward the first end 21. The single aperture 30 may be positioned 10 % or more, 25 % or more, 50 % or more, or 75 % or more toward the first end 21. The actual distance D30 of the aperture 30 from the midpoint 26 may be selected based on the size of the capsule 20. In some cases, the capsule 20 is a size 1, size 2, size 3, size 4, or size 5 capsule, preferably a size 3. The capsule 20 may have a length of about 11 mm, about 14 mm, about 16 mm, about 18 mm, or about 19 mm. The capsule side wall 25 may have a length of about 8 mm, about 10 mm, about 11 mm, about 12 mm, or about 14 mm. The single aperture 30 may be positioned 1 mm or more, 3 mm or more, or 5 mm or more toward the first end 21 of the capsule 20, measured from the midpoint 26. The single aperture 30 may be positioned 7 m or less, 6 mm or less, or 5 mm or less toward the first end 21 of the capsule 20, measured from the midpoint 26. The single aperture 30 may be within 2 mm, within 4 mm, within 6 mm, or within 10 mm of the first end 21 as measured from the first end 21 along a longitudinal axis A20 of the capsule 20.

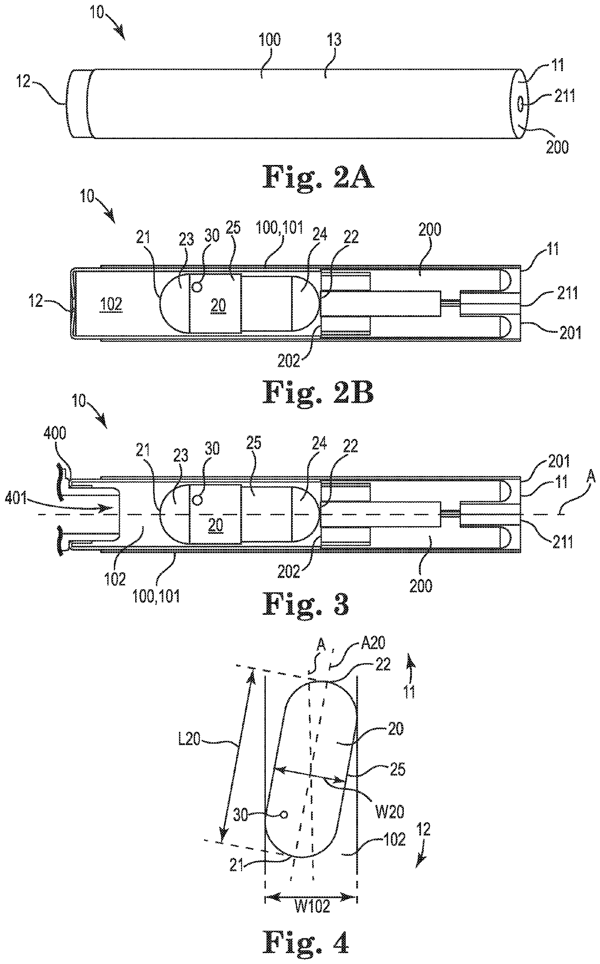

An inhaler article 10 containing the capsule 20 is shown schematically in FIGURES 2A, and 2B. The inhaler article 10 has a first, downstream end 11 and an opposing second, upstream end 12. A mouthpiece element 200 is disposed at the downstream end 11 of the inhaler article 10. The outside surface of the inhaler article 10 may be formed, at least in part, by a wrapper 13. The inhaler article 10 comprises an inhaler body 100 with a tubular side wall 101. The inhaler body 100 defines an interior that forms a receptacle 102 for housing the capsule 20. The inhaler body 100 further defines a longitudinal center axis A. The mouthpiece element 200 extends along the longitudinal center axis A. The mouthpiece element 200 may be at least partially inserted into the inhaler body 100. The mouthpiece element 200 has a downstream end 201 and an upstream end 202. The upstream end 202 of the mouthpiece element 200 is received in the interior of the inhaler body 100 and forms the downstream end of the capsule receptacle 102.

The first end 21 of the capsule 20 may be oriented toward the upstream end 12 of the body. The second end 22 may be oriented toward the downstream end 11 of the body. In some embodiments, the single aperture 30 extends through the tubular side wall 20 and is disposed closer to the first end 21 of the capsule 20 and the upstream end 12 of the inhaler article 10 than the second end 22 and the downstream end 11.

The inhaler article 10 may be used with a holder 400, as shown in FIGURE 3. The holder 400 may be configured to open the upstream end 12 of the inhaler article 10. The holder 400 may be configured to provide an air inlet 401 for the inhaler article 10. The holder 400 may further be configured to pierce the capsule 20. The holder 400 may be configured to make a single aperture 30 on the side wall 25 of the capsule 20. When a user draws air through the inhaler article to use the inhaler, air may flows from the air inlet 401, through the receptacle 102, entrain dry powder particles from inside the capsule 20, and flow through the mouthpiece element 200, and out the outlet 211. According to an embodiment, a dry powder may be inhaled from the capsule 20 at an airflow rate of 5 mL/min or less, or 2 mL/min or less.

The capsule receptacle 102 may have width (diameter) W102 that is less than the length L20 of the capsule 20 such that the capsule 20 maintains its orientation within the capsule receptacle 102. The first end 21 of the capsule 20 is oriented toward the upstream end 12, and the second end 22 of the capsule 20 is oriented toward the downstream end 11 of the inhaler 10. As shown in FIGURE 4, the capsule 20 may have room to move slightly (e.g., vibrate and rotate) but not enough to turn end over end. The capsule has a width (diameter) W20. The width (diameter) W102 of the capsule receptacle 102 may be 5 % to 25 % greater, 8 % to 22 % greater, or 10 % to 20 % greater than the width (diameter) W20 of the capsule 20.

### EXAMPLE

The performance of powder delivery of capsules having a single aperture at different locations was tested. Each of the capsules was a size 3 HPMC capsule (having a total length of 15.9 ± 0.3 mm) and contained 50 mg of powder containing nicotine salt and flavor particles. The apertures were made with a needle having a diameter of 0.8 mm.

Sample 1 was a capsule with a single aperture at the center of the first (upstream) end.

Sample 2 was a capsule with a single aperture at the side wall, positioned closer to the first (upstream) end. The aperture of Sample 2 was 13.6 mm from the downstream end, or 2.3 mm from the upstream end of the capsule.

Sample 3 was a capsule with a single aperture at the side wall, positioned closer to the second (downstream) end. The aperture of Sample 3 was 2.5 mm from the downstream end of the capsule.

The samples were tested by placing the capsule in an inhaler article capsule receptacle and drawing air through the inhaler article. The air draw pattern mimicked smoking a cigarette, drawing 80 mL or air for 2 sec (a single puff), 12 times. The amount of powder delivered over 12 puffs was measured. The results are shown in TABLE 1 below.

**TABLE 1. Powder delivery.**

| | Aperture Location | Powder Delivery, 12 Puffs |
|---|---|---|
| Sample 1 | Upstream end, center | 34.1 mg |
| Sample 2 | Side wall, near upstream end | 31.5 mg |
| Sample 3 | Side wall, near downstream end | 11.7 mg |

It was observed that a single aperture in the side wall near the upstream end delivered nearly the same amount of powder as an aperture positioned at the center of the upstream end. The difference in powder delivery between Sample 1 and Sample 2 was not statistically significant. It was surprisingly observed that a single aperture in the side wall near the upstream end delivered powder much more efficiently than a single aperture in the side wall near the downstream end.

## Claims

1. An inhaler article (10) comprising:
a body (100) comprising an upstream end (12) and a downstream end (11), a capsule receptacle (102), and an airflow path extending from the capsule receptacle to an opening at the downstream end; and
a capsule (20) disposed inside the capsule receptacle, the capsule comprising:
a first end (21) and a second end (22);
a tubular side wall (25) extending between the first and second ends;
only a single aperture (30) in the capsule, the single aperture extending through the tubular side wall; and
an inhalable powder (50) disposed inside the capsule,
wherein the first end of the capsule is oriented toward the upstream end of the body, and wherein the single aperture is disposed closer to the first end than the second end.

2. The inhaler article of claim 1, wherein each of the first and second ends comprises a hemisphere.

3. The inhaler article of any one of claims 1 to 2, wherein the capsule has a diameter, and the capsule receptacle has a diameter that is no more than 25 % of the capsule diameter.

4. The inhaler article of any one of claims 1 to 3, wherein the single aperture is within 2 mm to within 10 mm of the first end as measured along a longitudinal axis (A20) of the capsule.

5. The inhaler article of any one of claims 1 to 4, wherein the single aperture has an area of 0.2 mm² to 1.5 mm².

6. The inhaler article of any one of claims 1 to 5, wherein the inhalable powder comprises nicotine, preferably nicotine salt.

7. The inhaler article of any one of claims 1 to 6, wherein the inhalable powder contains nicotine particles with a mass median aerodynamic diameter particle size in a range of 0.5 µm to 10 µm.

8. The inhaler article of any one of claims 1 to 7, wherein the inhalable powder comprises flavor particles.

9. The inhaler article of any one of claims 1 to 8, wherein capsule is a polymeric capsule, preferably comprising hydroxypropylmethylcellulose.

10. The inhaler article of any one of claims 1 to 9, wherein the capsule is a size 1 to a size 4 capsule, or a size 2 to a size 3 capsule, preferably a size 3 capsule.

11. The inhaler article of any one of claims 1 to 10, wherein the body is constructed of cellulosic material, preferably paper.

12. The inhaler article of any one of claims 1 to 11, wherein the body comprises a tubular member constructed of cellulosic material, preferably paper.

13. The inhaler article of any one of claims 1 to 12, wherein the upstream end of the tubular body forms a distal end of the capsule receptacle.

14. The inhaler article of any one of claims 1 to 13, wherein the inhaler article comprises a mouthpiece element (200) received at the downstream end of the body.

15. The inhaler article of claim 14, wherein the mouthpiece element defines a downstream end of the capsule receptacle.

## Patentansprüche

1. Inhalatorartikel (10), aufweisend:
einen Körper (100), aufweisend ein vorgelagertes Ende (12) und ein nachgelagertes Ende (11), eine Kapselaufnahme (102) und einen sich von der Kapselaufnahme zu einer Öffnung an dem nachgelagerten Ende erstreckenden Luftstrompfad; und
eine Kapsel (20), die innerhalb der Kapselaufnahme angeordnet ist, wobei die Kapsel aufweist:
- ein erstes Ende (21) und ein zweites Ende (22);
eine rohrförmige Seitenwand (25), die sich zwischen dem ersten und dem zweiten Ende erstreckt;
nur eine einzelne Öffnung (30) in der Kapsel, wobei sich die einzelne Öffnung durch die rohrförmige Seitenwand erstreckt; und ein inhalierbares Pulver (50), das innerhalb der Kapsel angeordnet ist,
wobei das erste Ende der Kapsel auf das vorgelagerte Ende des Körpers ausgerichtet ist, und wobei die einzelne Öffnung näher an dem ersten Ende als an dem zweiten Ende angeordnet ist.

2. Inhalatorartikel nach Anspruch 1, wobei sowohl das erste als auch das zweite Ende eine Halbkugel umfasst.

3. Inhalatorartikel nach einem der Ansprüche 1 bis 2, wobei die Kapsel einen Durchmesser aufweist und die Kapselaufnahme einen Durchmesser aufweist, der nicht mehr als 25 % des Kapseldurchmessers beträgt.

4. Inhalatorartikel nach einem der Ansprüche 1 bis 3, wobei die einzelne Öffnung innerhalb von 2 mm bis innerhalb von 10 mm von dem ersten Ende, gemessen entlang einer Längsachse (A20) der Kapsel, liegt.

5. Inhalatorartikel nach einem der Ansprüche 1 bis 4, wobei die einzelne Öffnung eine Fläche von 0,2 mm² bis 1,5 mm² aufweist.

6. Inhalatorartikel nach einem der Ansprüche 1 bis 5, wobei das inhalierbare Pulver Nikotin, bevorzugt Nikotinsalz, umfasst.

7. Inhalatorartikel nach einem der Ansprüche 1 bis 6, wobei das inhalierbare Pulver Nikotinpartikel mit einem mittleren aerodynamischen Massendurchmesser der Partikelgröße in dem Bereich von 0,5 µm bis 10 µm enthält.

8. Inhalatorartikel nach einem der Ansprüche 1 bis 7, wobei das inhalierbare Pulver Geschmackspartikel umfasst.

9. Inhalatorartikel nach einem der Ansprüche 1 bis 8, wobei die Kapsel eine Polymerkapsel ist, die bevorzugt Hydroxypropylmethylcellulose aufweist.

10. Inhalatorartikel nach einem der Ansprüche 1 bis 9, wobei die Kapsel eine Kapsel der Größe 1 bis 4 oder eine Kapsel der Größe 2 bis 3, bevorzugt eine Kapsel der Größe 3, ist.

11. Inhalatorartikel nach einem der Ansprüche 1 bis 10, wobei der Körper aus Cellulosematerial, bevorzugt Papier, hergestellt ist.

12. Inhalatorartikel nach einem der Ansprüche 1 bis 11, wobei der Körper ein röhrenförmiges Element aufweist, das aus Cellulosematerial, bevorzugt Papier, hergestellt ist.

13. Inhalatorartikel nach einem der Ansprüche 1 bis 12, wobei das vorgelagerte Ende des rohrförmigen Körpers ein distales Ende der Kapselaufnahme bildet.

14. Inhalatorartikel nach einem beliebigen der Ansprüche 1 bis 13, wobei der Inhalatorartikel ein Mundstückelement (200) aufweist, das an dem nachgelagerten Ende des Körpers aufgenommen ist.

15. Inhalatorartikel nach Anspruch 14, wobei das Mundstückelement ein nachgelagertes Ende der Kapselaufnahme definiert.

## Revendications

1. Article inhalateur (10) comprenant :
un corps (100) comprenant une extrémité amont (12) et une extrémité aval (11), un réceptacle de capsule (102), et un trajet d'écoulement d'air s'étendant du réceptacle de capsule à une ouverture au niveau de l'extrémité aval ; et
une capsule (20) disposée à l'intérieur du réceptacle de capsule, la capsule comprenant :
une première extrémité (21) et une deuxième extrémité (22) ;
une paroi latérale tubulaire (25) s'étendant entre les première et deuxième extrémités ;
un unique orifice (30) dans la capsule, l'unique orifice s'étendant à travers la paroi latérale tubulaire ; et
une poudre inhalable (50) disposée à l'intérieur de la capsule,
dans lequel la première extrémité de la capsule est orientée vers l'extrémité amont du corps, et dans lequel l'unique orifice est disposé plus près de la première extrémité que de la deuxième extrémité.

2. Article inhalateur selon la revendication 1, dans lequel chacune des première et deuxième extrémités comprend un hémisphère.

3. Article inhalateur selon l'une quelconque des revendications 1 à 2, dans lequel la capsule a un diamètre, et le réceptacle de capsule a un diamètre qui n'est pas supérieur à 25 % du diamètre de la capsule.

4. Article inhalateur selon l'une quelconque des revendications 1 à 3, dans lequel l'unique orifice est à moins de 2 mm jusqu'à moins de 10 mm de la première extrémité, comme mesurée le long d'un axe longitudinal (A20) de la capsule.

5. Article inhalateur selon l'une quelconque des revendications 1 à 4, dans lequel l'unique orifice a une aire de 0,2 mm² à 1,5 mm².

6. Article inhalateur selon l'une quelconque des revendications 1 à 5, dans lequel la poudre inhalable comprend de la nicotine, de préférence un sel de nicotine.

7. Article inhalateur selon l'une quelconque des revendications 1 à 6, dans lequel la poudre inhalable contient des particules de nicotine avec une taille de particule, diamètre aérodynamique moyen en masse, dans une plage de 0,5 µm à 10 pm.

8. Article inhalateur selon l'une quelconque des revendications 1 à 7, dans lequel la poudre inhalable comprend des particules d'arôme.

9. Article inhalateur selon l'une quelconque des revendications 1 à 8, dans lequel la capsule est une capsule polymère, comprenant de préférence de l'hydroxypropylméthylcellulose.

10. Article inhalateur selon l'une quelconque des revendications 1 à 9, dans lequel la capsule est une capsule de taille 1 à taille 4, ou une capsule de taille 2 à taille 3, de préférence une capsule de taille 3.

11. Article inhalateur selon l'une quelconque des revendications 1 à 10, dans lequel le corps est constitué d'une matière cellulosique, de préférence de papier.

12. Article inhalateur selon l'une quelconque des revendications 1 à 11, dans lequel le corps comprend un membre tubulaire constitué d'une matière cellulosique, de préférence de papier.

13. Article inhalateur selon l'une quelconque des revendications 1 à 12, dans lequel l'extrémité amont du corps tubulaire forme une extrémité distale du réceptacle de capsule.

14. Article inhalateur selon l'une quelconque des revendications 1 à 13, dans lequel l'article inhalateur comprend un élément d'embout buccal (200) reçu au niveau de l'extrémité aval du corps.

15. Article inhalateur selon la revendication 14, dans lequel l'élément d'embout buccal définit une extrémité aval du réceptacle de capsule.
